Europäisches Patentamt

European Patent Office

Office européen des brevets

(11) Publication number: **0 136 362**
**B1**

(12) **EUROPEAN PATENT SPECIFICATION**

(45) Date of publication of patent specification: **19.12.90**

(51) Int. Cl.⁵: **G 01 N 27/327**

(21) Application number: **84901016.0**

(22) Date of filing: **06.03.84**

(86) International application number:
**PCT/JP84/00087**

(87) International publication number:
**WO 84/03562 13.09.84 Gazette 84/22**

(54) **BIOSENSOR.**

(30) Priority: **11.03.83 JP 40885/83**
**19.07.83 JP 132403/83**

(43) Date of publication of application:
**10.04.85 Bulletin 85/15**

(45) Publication of the grant of the patent:
**19.12.90 Bulletin 90/51**

(84) Designated Contracting States:
**DE FR GB**

(56) References cited:
**EP-A-0 096 095**
**EP-A-0 121 385**
**GB-A-1 318 815**
**JP-A-5 510 584**
**JP-A-5 598 347**
**JP-A-5 798 853**
**JP-A-57 106 850**

(73) Proprietor: **MATSUSHITA ELECTRIC INDUSTRIAL CO., LTD.**
**1006, Oaza Kadoma**
**Kadoma-shi, Osaka-fu, 571 (JP)**

(72) Inventor: **KAWAGURI, Mariko**
**19-8, Senrioka-kami Suita-shi**
**Osaka-fu 565 (JP)**
Inventor: **NANKAI, Shiro**
**50-12, Nasuzukuri 4-chome Hirakata-shi**
**Osaka-fu 573 (JP)**
Inventor: **IIJIMA, Takashi**
**35-26, Yamanoue-nishimachi Hirakata-shi**
**Osaka-fu 573 (JP)**

(74) Representative: **Eisenführ, Speiser & Strasse**
**Martinistrasse 24**
**D-2800 Bremen 1 (DE)**

Courier Press, Leamington Spa, England.

## Description

The present invention relates to a bio-sensor for measuring the substrate concentration of a liquid sample, which makes it possible to determine the particular component of various biological samples with ease, rapidity and high precision.

In recent years, various kinds of bio-sensor based on the specific catalytic action of an enzyme have been developed, and particularly, their application in clinical inspections is being tried. Since inspection items and the number of specimens are increasing, bio-sensors which enable rapid and high-precision measurement are demanded.

A glucose sensor will be taken as an example. In view of the remarkable increase of diabetes, for measurement and control of blood sugar in blood, there is a demand for sensors applicable to tests on whole blood, because it takes a very long time to centrifuge blood and measure the separated plasma as conventionally carried out. A simple sensor is available. Like the test paper used for urinalysis, this sensor comprises a stick-form support and a carrier put on the support containing an enzyme which will react with glucose only and a dye which will change itself on enzyme reaction or by the product of the reaction. Blood is added to the carrier, and a change in the dye after a definite period of time is measured visually or optically. But, the measurement is largely disturbed by dyes in blood, so that its precision is low.

From GB—A—1318815 a bio-sensor for measuring the substrate concentration of a liquid sample is known, which sensor comprises an insulating substrate plate provided with an electrode system containing at least a working electrode and a counter electrode, the electrode system being covered by a substrate permeable to liquid and containing an enzyme capable of inducing a substrate reaction, which is electrochemically detectable by means of the electrode system, as indicated in the pre-characterizing portion of appended claim 1. This bio-sensor uses a semi-permeable membrane, which contains immobilized urease. After contacting the sensor with an urea-containing sample, the liquid diffuses into the membrane, where the urea is broken down by the urease into ammonia and carbon dioxide. Ammonia reaction with water leads to the formation of ions, the presence of which modifies the conductivity. This conductivity change is detected by the electrode.

This sensor requires the presence of an additional electrode to compensate the conductivity change. The sensor shows relatively long reaction times, both due to the semi-permeable character of the membrane and the immobilization of the enzyme. High substrate concentrations cannot be reliably determined.

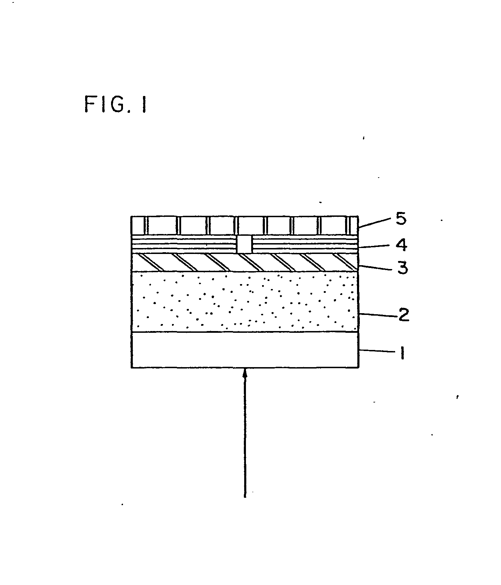

The multi-layer analytical carrier as shown in Fig. 1 was proposed (Japanese Utility Model Opening (Kokai) No. 178495/1979). This carrier has a laminated structure wherein a reagent layer 2, a developing layer 3, a water-proof layer 4 and a filter layer 5 are placed one upon another in this order on a transparent support 1. When a blood sample is dropped on to the carrier, the blood is first freed from its solid components such as red blood cells, blood platelets, etc. on the filter layer 5, uniformly diffuses into the developing layer 3 through small pores in the water-proof layer 4 and comes into reaction in the reagent layer 2. After completion of the reaction, light is passed through the transparent support in the arrow direction to measure the substrate concentration spectroanalytically. Although this carrier is complicated in structure as compared with the conventional sample stick-form carrier, an improvement in precision was attained by removing blood cells, etc. Nevertheless the penetration and reaction of blood take a long time, so that the water-proof layer 4 is needed for preventing the sample from drying, and it is necessary to incubate at high temperatures for accelerating the reaction, which requires complicated apparatus and carrier.

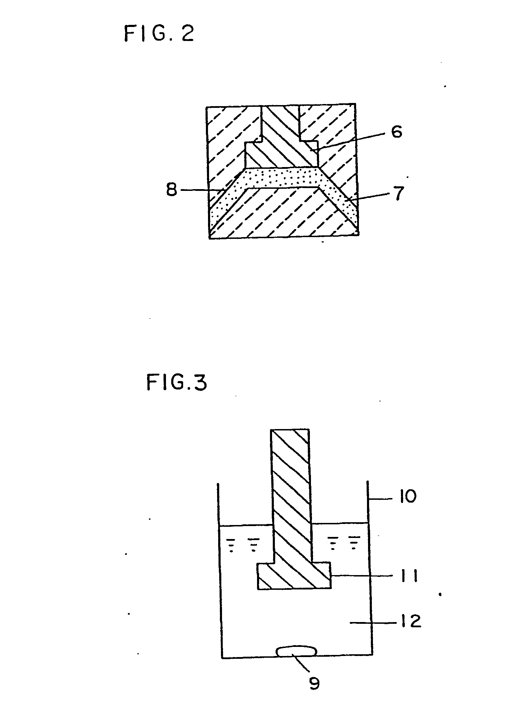

Thus, an electrode method combining an enzyme reaction with an electrode reaction was developed as a simple method of good precision. Since there is no disturbance of colored substances in the electrode method, the liquid sample can be used as it is without necessity for pre-treatment, and therefore, the measurement became simple and precision was improved. A glucose sensor will be taken as an example. A flow-type sensor was developed as shown in Fig. 2, in which a liquid sample, e.g. blood or urine, is added to the sensor while applying a definite voltage to a glucose oxidase immobilized electrode 6 and passing a buffer solution 7 through a conduit 8 made of insulating materials such as acrylic resin; glucose in the sample reacts with the immobilized glucose oxidase to produce hydrogen peroxide which is then oxidized at the electrode 6 to generate a current; and the glucose concentration of the sample can be detected by measuring the strength of the current. The sensor of this type enables as many specimens as 200 to 300 per hour to be measured with rapidity and high precision, but the apparatus becomes large in size. The so-called batch-type sensor as shown in Fig. 3 was thus developed, in which a glucose oxidase immobilized electrode 11 is placed in a vessel 10 which is then filled with a buffer solution 12, and a liquid sample is added to the solution while stirring with a stirrer. Sensors of this kind are known from Patent Abstracts of Japan, Vol. 4, No. 39 and Vol. 6, No. 188, and corresponding publications JP—A—55-10584 and JP—A—57-98853. The size of apparatus could be made fairly small by using this type, but there occurred problems that a stirrer is essential, and that foaming and turbulent flow are caused by stirring to exert an adverse effect on the precision. Also, there was a necessity to exchange the buffer solution and sometimes wash the electrode, and besides, because of the liquid sample being diluted with the buffer solution, precision was required for the amounts of buffer solution and liquid sample.

Thus, for simple measurement, a dry measurement-form sensor requiring no stirring apparatus is demanded, providing high precision at short reaction times.

The bio-sensor of the present invention is composed of an insulating substrate plate and an electrode system comprising a working electrode and a counter electrode put thereon, said system being covered with a porous substrate comprising at least one layer containing oxidoreductase and the sensor further containing an electron acceptor, both the oxidoreductase and the electron acceptor being soluble in the liquid sample. This sensor is intended to measure the concentration of substrates, e.g. urine sugar and blood sugar, by electrochemically detecting a reduced electron acceptor produced when it is impregnated with a liquid sample to carry out enzyme reaction, and also it is intended to measure the glucose concentration of fruit juices.

By using the bio-sensor of the present invention, measurement can be carried out by merely adding a liquid sample directly to the porous substrate without using a buffer solution. Also, since the sample need not be diluted, there is no necessity to determine the sample and besides a trace amount of the particular component of the sample can be measured with simplicity and high sensitivity.

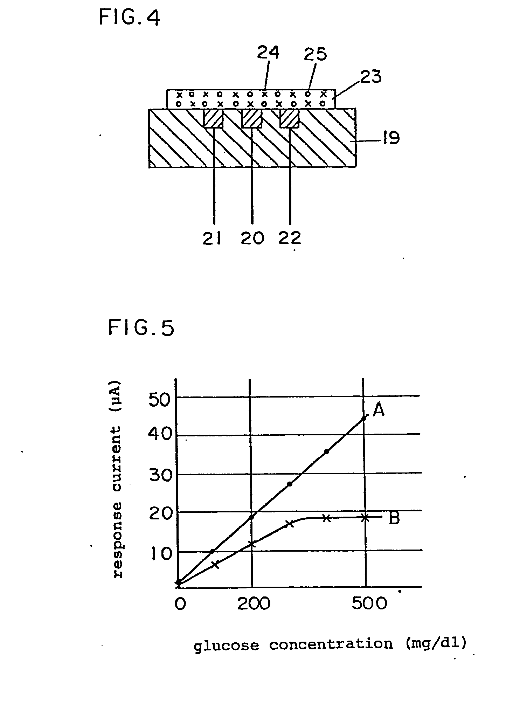

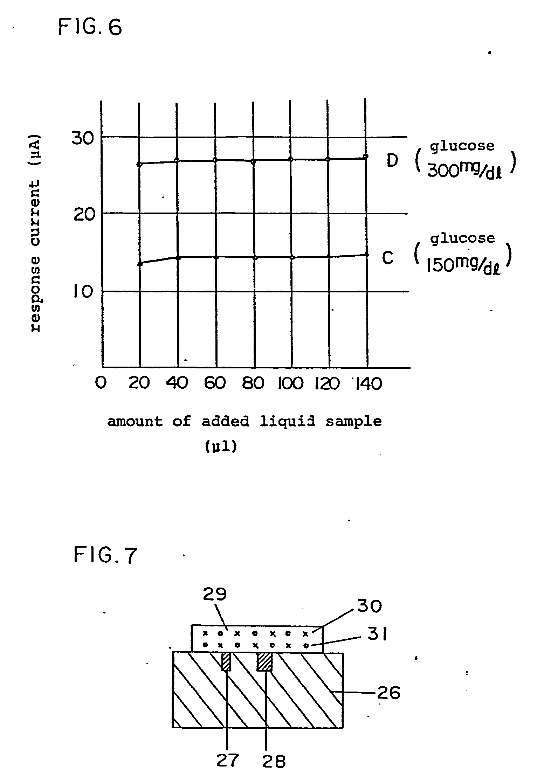

Fig. 1 is a typical view illustrating one embodiment of the conventionally used glucose sensors. Figs. 2 and 3 are typical views illustrating the conventional glucose sensor using an immobilized enzyme electrode. Fig. 4 is a typical view illustrating one embodiment of the bio-sensor of the present invention. Figs. 5 and 6 show the response characteristics of the bio-sensor shown in Fig. 4. Figs. 7, 8 and 9 are typical views illustrating other embodiments of the bio-sensor of the present invention.

Example 1

Fig. 4 is a typical view illustrating one embodiment of a glucose sensor. Pieces of platinum wire of 1 mm in diameter were buried in an insulating substrate plate 19 made of a polyvinyl chloride resin to prepare a working electrode 20, counter electrode 21 and reference electrode 22, and nylon non-woven fabric 23 was put on the foregoing electrode system so as to cover the system. This nylon non-woven fabric 23 was prepared as follows: 100 mg of glucose oxidase and 150 mg of potassium ferricyanide are dissolved in 1 ml of a phosphate buffer solution (pH 5.6), and nylon non-woven fabric is impregnated with the solution thus prepared and dried at room temperature.

A standard glucose solution, a liquid sample, was added to the nylon non-woven fabric 23 and after allowing the solution to well penetrate into the fabric, voltage was applied to the working electrode 20 with the reference electrode 22 as standard and varied at a rate of 0.1 V/sec so that it drew a saw-toothed line between 0 V and +0.5 V. When the added glucose is oxidized by glucose oxidase 24 carried on the nylon non-woven fabric 23, potassium ferricyanide 25 is reduced by enzyme/dye conjugation reaction to produce potassium ferrocyanide. On oxidizing the potassium ferrocyanide by sweeping voltage applied to the working electrode 20, anodic current flows. This anodic current is directly proportional to the amount of dye changed, and when the dye is present in sufficient amounts, since said amount corresponds to the substrate concentration, the concentration of glucose, the substrate, can be detected by measuring the strength of the anodic current. The relationship between the strength of the peak current obtained and the concentration of glucose added showed a very good rectilinearity in the range of up to 500 mg/dl as shown by A in Fig. 5. The nylon non-woven fabric 23 was exchanged at every measurement, but reproducibility was good. Also, the amount of the standard glucose solution added was varied from 20 µl to 140 µl, but a definite value was obtained independently of the amount of the solution as shown by C and D in Fig. 6 when the glucose concentration was definite.

Example 2

The electrode system used in Example 1 was covered at the upper surface with nylon non-woven fabric carrying glucose oxidase only, and then measurement was carried out in the same manner as in Example 1 with dropwise addition of the standard glucose solution. In this system, oxygen contained in air and the liquid sample is used as the electron acceptor; when glucose reacts with glucose oxidase, hydrogen peroxide is produced in an amount corresponding to the glucose concentration and oxidized at the surface of the electrode; and the strength of anodic current generated by the oxidation is measured to obtain the glucose concentration. The peak of the current obtained, as shown by B in Fig. 5, showed a rectilinear rise up to 300 mg/dl but little or no rise at higher concentrations than this. The reason for this may be insufficient supply of oxygen when the substrate concentration is high. But measurement was possible at low concentrations of the substrate.

Example 3

Fig. 7 is a typical view illustrating another embodiment of the glucose sensor of the present invention. As shown in Example 1, measurement can stably be carried out if a three-electrode system comprising a working electrode, a counter electrode and a reference electrode is used, but it can also be carried out with a two-electrode system comprising a working electrode and a counter electrode. Platinum was buried in an insulating substrate plate 26 made of a polyvinyl chloride resin to prepare a working electrode 27 and counter electrode 28. To stabilize the potential, the area of the counter electrode 28 was made at least more than two times as large as that of the working electrode 27. In the same manner as in Example 1, this electrode system was covered at the upper surface with nylon non-woven fabric 29 carrying glucose oxidase 30 and potassium ferricyanide 31, and measurement was carried out with dropwise addition of the standard glucose solution. It was found that the reproducibility was slightly poorer than in Example 1, but that glucose concentrations of up to 500 mg/dl could be measured. A silver/silver chloride (Ag/AgCl) counter electrode was used in place of the platinum one 28, and it was found that the potential was stabilized, and that measurement could be carried out with good reproducibility if the area of this electrode was made equal to that of the electrode 27.

Example 4

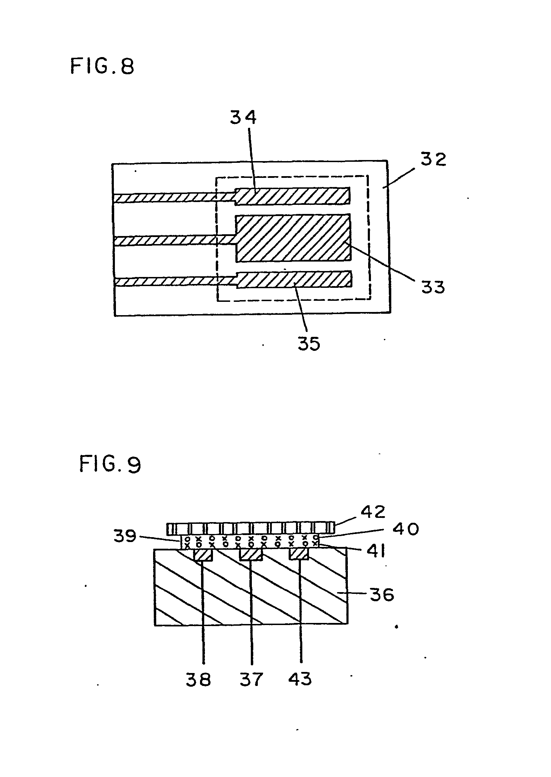

Fig. 8 is a sensor comprising an insulating substrate plate 32 made of a polyvinyl chloride resin and a working electrode 33, counter electrode 34 and reference electrode 35 in a thin film form which were produced from platinum on the substrate plate by spattering. On these electrodes was placed the same nylon non-woven fabric carrying the enzyme and oxidizing dye as used in Example 1 so as to cover the region shown by the dotted line. Thereafter, response to the standard glucose solution was measured in the same manner as in Example 1 to obtain the same good response as in the electrode system in Fig. 4. Next, measurement was repeated using the standard glucose solution of the same concentration as well as the electrodes and nylon non-woven fabric newly exchanged at every measurement, and as a result, a very good reproducibility was obtained. Consequently, if the electrodes are produced by spattering or vacuum deposition as in this example, electrodes having the same response characteristics and any desired shape can be produced in large amounts, and by covering the electrodes with a porous substrate carrying enzyme and oxidizing dye, high-performance bio-sensors which can be disposed after use, can be provided.

Example 5

Fig. 9 is a typical view illustrating one embodiment of a glucose sensor. Platinum is buried in an insulating substrate plate 36 made of a polyvinyl chloride resin to prepare a working electrode 37, counter electrode 38 and reference electrode 43. Nylon non-woven fabric 39 is placed on the electrode system comprising these electrodes so as to cover the system. This nylon non-woven fabric 39 is prepared through the same dissolution, impregnation and drying steps as in Example 1, and it carries glucose oxidase 40 (oxidoreductase) and potassium ferricyanide 41 (oxidizing dye), which works in conjugation with oxidoreductase, in a dry form. On the upper surface of the fabric 39 is placed a filter layer 42 comprising porous polycarbonate (pore diameter, 1 μm).

On adding blood (whole blood) to this sensor, large particles in blood such as red blood cells were filtered off on the filter layer 42, and glucose in the blood came into reaction at the reaction layer comprising the nylon non-woven fabric 39 in the same manner as in Example 1, whereby the glucose concentration could be detected by the electrode system. That is, from the strength of the current obtained, the glucose concentration could be detected based on a calibration curve previously prepared with a standard glucose solution. The current strength and glucose concentration showed a good correlation as compared with the conventionally used types of glucose sensor. The reaction layer comprising the enzyme and oxidizing dye and the filter layer were exchanged at every measurement, but the reproducibility was good in either sample of standard glucose solution and blood. Also, the amount of blood added was varied from 20 μl to 140 μl, but the amount of glucose showed a definite value independently of said amount since the dye and enzyme were present in sufficient amounts.

By using a porous substrate of polycarbonate as the filter layer 42, blood cells and viscous substances present in blood could previously be removed, and as a result, stains on the electrode could be decreased. Without the filter layer, blood cells adhered to the electrode during a long-term use, lowering the strength of current obtained, so that there was the necessity to wash the electrode with alcohol. But, by using the filter layer, it became possible to maintain the response with good reproducibility by merely washing the electrode with water. Also, it was found that the porous substrate which was useful to the above object was a one having a pore diameter within about 3 μm. Further when the porous substrate of polycarbonate was used after dipping in a 1% aqueous solution of surface active agent, e.g. polyethylene glycol alkylphenyl ether (trade name: Triton X) and drying, filtration of blood became very fast with further improved reproducibility. Hitherto, there was a problem that, since blood is fairly viscous, its filtration rate is low. But, by using a filter layer previously treated with surface active agents, filtration became fast and also reaction with enzyme and dye proceeded rapidly and uniformly to come to an end in as short a time as only about one minute after addition of a test sample. When the surface active agent was not used, it took about 1.5 minutes for the reaction to come to an end after addition of blood, and therefore the effect of the surface active agent to accelerate the measurement was large.

As the surface active agent, polyoxyethylene glycerin fatty acid esters, polyoxyethylene alkyl ethers, polyethylene glycol fatty acid esters and the like can be used in addition to the foregoing example. By previous treatment of not only the filter layer but also the dye and enzyme with the surface active agent, the rates of filtration and penetration became fast, accelerating the measurement.

The reaction layer comprising the oxidizing dye and enzyme is preferably a hydrophilic porous membrane so that it can absorb the liquid sample rapidly to advance the enzyme reaction. For example, when filter paper, non-woven fabric of pulp, porous substrate of ceramic or glass, etc. were used in addition to the nylon non-woven fabric, penetration of the liquid sample was quick and uniform, and reproducibility was also good. Further, the rate of penetration of the sample into the above porous membrane was larger in the membrane pre-treated with the foregoing surface active agent than in the untreated membrane. The filter layer having an effect to accelerate the measurement may be placed on the electrode or the upper surface of the reaction layer. Penetration of the liquid sample was fastest when the filter layer was placed below the reaction layer, and the reaction time was also short. When the filter layer was placed on the reaction layer, there resulted an advantage in that the reaction proceeded smoothly and a high precision was obtained since the solid component of blood was filtered off so that there was no disturbance by blood cells, etc. in the reaction layer. For the filter layer, use of non-woven fabrics, chemical fibers, paper (filter paper), etc. was thought of. The solid components of blood cells could completely be filtered off by using membrane filters having a pore diameter of less than about 2—3 $\mu$m. Also, filtration could be carried out stepwise by laminating the membrane filter layer with the foregoing non-woven fabric, chemical fiber, paper or the like.

In the above sensor, various methods could be employed to carry glucose oxidase (enzyme) and potassium ferricyanide (dye). For example, it was possible to laminate two pieces of nylon non-woven fabric carrying the enzyme and dye, respectively. In carrying potassium ferricyanide on nylon non-woven fabric 39, potassium ferricyanide crystals on the fabric became much finer and more soluble in liquids by hot air-drying nylon non-woven fabric impregnated with a potassium ferricyanide solution than by drying the fabric at room temperature. The potassium ferricyanide crystal on the fabric became further finer when the nylon non-woven fabric impregnated with a potassium ferricyanide solution was dipped in an organic solvent having a great solubility in water, for example ethanol, and vacuum-dried. By carrying potassium ferricyanide in a finely pulverized form, even a highly viscous sample such as blood completed the reaction within one minute with an improved reproducibility. Since the enzyme is poorly resistant to heat, etc., it was dried at room temperature after being dipped in the above organic solvent. Also, when both the enzyme and oxidizing dye are carried on one piece of porous substrate, the dye and enzyme could be carried in a very soluble form by dipping the body in ethanol and vacuum-drying.

Example 6

In Example 5, a porous membrane such as nylon non-woven fabric was used as a porous substrate, but in addition to this, inorganic carriers such as $SiO_2$ also may be used in the form of a porous substrate produced by press-molding. When $SiO_2$ was pressure-molded into a size of 7 mm (diameter)$\times$1 mm (thickness) by 490 N/mm$^2$ (5 t/cm$^2$) pressure, impregnated with an aqueous solution containing glucose oxidase and potassium ferricyanide and then dried, penetration of blood into this molded $SiO_2$ was a little slower than in the nylon non-woven fabric. Since, however, the porous membrane of polycarbonate also can be produced at the same time by pressure-molding, the filter layer and reaction layer could integrally be molded to simplify their production. Further, it was also possible to finely pulverize glucose oxidase and potassium ferricyanide and after mixing and adding a small amount of $SiO_2$, to pressure-mold the mixture. In this case, since the glucose oxidase and potassium ferricyanide rapidly dissolve in blood to form a uniform mixture, the reaction became very fast.

Example 7

In Example 5, blood was filtered using a filter layer treated with a surface active agent. In this Example, however, this treated filter layer was further impregnated with 10 mg/ml of a sodium fluoride (anticoagulant) solution and dried, and the procedure of Example 5 was repeated but using the filter layer thus obtained. As a result, blood could be filtered in only ten seconds without coagulation, and besides measurement was completed in as short a time as only 50 seconds after addition of blood. Thus, this method made a great contribution to acceleration of measurement.

As the anticoagulant, sodium fluoride is suitable because it is stable and simple in handling. But, heparin, sodium citrate, ethylenediamine tetraacetic acid, etc. were also useful to carry out blood filtration rapidly.

As the dye, potassium ferricyanide used in the foregoing Examples is suitable because it reacts stably, but p-benzoquinone is suitable for acceleration because it is fast in reaction rate. Further, 2,6-dichlorophenolindophenol, methylene blue, phenazine methosulfate, potassium $\beta$-naphthoquinone-4-sulfonate, etc. may also be used.

The sensors in the foregoing Examples can be applied to not only glucose but also systems in which oxidoreductase takes part such as alcohol sensors, cholesterol sensors and the like. Glucose oxidase was used as the oxidoreductase, but other enzymes such as alcohol oxidase, xanthine oxidase, cholesterol oxidase, etc. may also be used.

Possibility of application to industry:

The bio-sensor of the present invention makes it possible to measure the particular component in various biological samples with rapidity, high precision and simplicity, so that its utility value in clinical inspection is very large.

List of symbols in the drawing:

| 1 | Support |
|---|---|
| 2 | Reagent layer |
| 3 | Developing layer |
| 4 | Water-proof layer |
| 5, 42 | Filter layer |
| 6, 11 | Immobilized electrode |
| 7, 12 | Buffer solution |
| 8 | Conduit |
| 9 | Stirrer |
| 10 | Vessel |
| 20, 27, 33, 37 | Working electrode |
| 21, 28, 34, 38 | Counter electrode |
| 22, 35, 43 | Reference electrode |
| 23, 29, 39 | Non-woven fabric |
| 24, 30, 40 | Glucose oxidase |
| 25, 31, 41 | Potassium ferricyanide |
| 26, 32, 36 | Insulating substrate plate |

## Claims

1. A bio-sensor for measuring the substrate concentration of a liquid sample, comprising an insulating substrate plate (19; 26; 32; 36) provided with an electrode system containing at least a working electrode (20; 27; 33; 37) and a counter electrode (21; 28; 34; 38), the electrode system being covered by a substrate (23; 29; 39) permeable to liquid and containing an enzyme (24; 30; 40) capable of inducing a substrate reaction which is electrochemically detectable by means of the electrode system, characterized in that the substrate (23; 29; 39) is porous and contains an oxidoreductase (24; 30; 40), the sensor further containing an electrode acceptor (25; 31; 41), and both the oxidoreductase and the electron acceptor being soluble in the liquid sample.

2. A bio-sensor according to claim 1, characterized in that the enzyme (24; 30; 40) and/or the electron acceptor (25; 31; 41) is in the form of finely divided particles.

3. A bio-sensor according to claim 1, characterized in that said porous substrate carries both said oxidoreductase (24; 30; 40) and electron acceptor (25; 31; 41).

4. A bio-sensor according to claim 1, characterized in that said working electrode (20; 27; 33; 37) comprises platinum and said counter electrode (21; 28; 34; 38) comprises platinum or silver/silver chloride.

5. A bio-sensor according to claim 1, characterized in that said electrode system is composed of a working electrode (20; 27; 33; 37), a counter electrode (21; 28; 34; 38) and a reference electrode (22; 35; 43).

6. A bio-sensor according to claim 1, characterized in that said porous substrate comprises a hydrophilic porous membrane.

7. A bio-sensor according to claim 1, characterized in that said electron acceptor is oxygen.

8. A bio-sensor according to claim 1, characterized in that said enzyme (24; 30; 40) is one member selected from the group consisting of glucose oxidase, alcohol oxidase, xanthine oxidase and cholesterol oxidase.

9. A bio-sensor according to claim 1, characterized in that, as said electron acceptor, an oxidizing dye (25; 31; 41) working in conjugation with said oxidoreductase, is used.

10. A bio-sensor according to claim 9, characterized in that, as said oxidizing dye (25; 31; 41) working in conjugation with the oxidoreductase, one member selected from the group consisting of potassium ferricyanide, p-benzoquinone, 2,6-dichlorophenolindophenol, methylene blue, phenazine methosulfate and potassium β-naphthoquinone-4-sulfonate is used.

11. A bio-sensor according to claim 9, characterized in that said oxidoreductase is glucose oxidase and said electron acceptor is potassium ferricyanide.

12. A bio-sensor according to claim 6, characterized in that at least the oxidoreductase is carried on said porous membrane in a dry state.

13. A bio-sensor according to claim 6, characterized in that said porous membrane (42) has a pore diameter of not more than 3 μm.

14. A bio-sensor according to claim 6, characterized in that said porous substrate was previously treated with a surface active agent.

15. A bio-sensor according to claim 6, characterized in that an anticoagulant is carried on said porous membrane (42).

## Patentansprüche

1. Biosensor zur Messung der Substratkonzentration einer Flüssigkeitsprobe, bestehend aus einer isolierenden Substratplatte (19; 26; 32; 36), die mit einem Elektrodensystem mit zumindest einer Arbeitselektrode (20; 27; 33; 37) und einer Gegenelektrode (21; 28; 34; 38) versehen ist, wobei das Elektrodensystem von einem Substrat (23; 29; 39) bedeckt ist, das Flüssigkeit aufnehmen kann und ein Enzym (24; 30; 40) enthält, welches zur Induzierung einer mittels des Elektrodensystems elektrochemisch nachweisbaren Substratreaktion fähig ist, dadurch gekennzeichnet, daß das Substrat (23; 29; 39) porös ist und eine Oxidoreduktase (24; 30; 40) enthält, wobei der Sensor weiterhin einen Elektronenakzeptor (25; 31; 41) aufweist, und sowohl die Oxidoreduktase als auch der Elektronenakzeptor in der Flüssigkeitsprobe löslich sind.

2. Biosensor nach Anspruch 1, dadurch gekennzeichnet, daß das Enzym (24; 30; 40) und/oder der Elektronenakzeptor (25; 31; 41) in Form von fein verteilten Teilchen vorliegt.

3. Biosensor nach Anspruch 1, dadurch gekennzeichnet, daß das poröse Substrat sowohl die Oxidoreduktase (24; 30; 40) als auch den Elektronenakzeptor (25; 31; 41) trägt.

4. Biosensor nach Anspruch 1, dadurch gekennzeichnet, daß die Arbeitselektrode (20; 27; 33; 37) aus Platin und die Gegenelektrode (21; 28; 34; 38) aus Platin oder Silber/Silberchlorid besteht.

5. Biosensor nach Anspruch 1, dadurch gekennzeichnet, daß das Elektrodensystem sich aus einer Arbeitselektrode (20; 27; 33; 37), einer Gegenelektrode (21; 28; 34; 38) und einer Bezugselektrode (22, 35; 43) zusammensetzt.

6. Biosensor nach Anspruch 1, dadurch gekennzeichnet, daß das poröse Substrat aus einer hydrophilen porösen Membran besteht.

7. Biosensor nach Anspruch 1, dadurch gekennzeichnet, daß es sich bei dem Elektronenakzeptor um Sauerstoff handelt.

8. Biosensor nach Anspruch 1, dadurch gekennzeichnet, daß das Enzym (24; 30; 40) aus der aus Glukoseoxidase, Alkoholoxidase, Xanthinoxidase und Cholesterinoxidase bestehenden Gruppe ausgewählt ist.

9. Biosensor nach Anspruch 1, dadurch gekennzeichnet, daß als Elektronenakzeptor ein Oxidationsfärbemittel (25; 31; 41) das mit der Oxidoreduktase zusammenwirkt, verwendet wird.

10. Biosensor nach Anspruch 9, dadurch gekennzeichnet, daß das Oxidationsfärbemittel (25; 31; 41), das mit der Oxidoreduktase zusammenwirkt, aus der aus Kaliumferrizyanid, p-Benzochinon, 2,6-Dichlorophenolindophenol, Methylenblau, Phenazinmethosulfat und Kalium β-Naphtochinon-4-sulfonat bestehenden Gruppe ausgewählt wird.

11. Biosensor nach Anspruch 9, dadurch gekennzeichnet, daß die Oxidoreduktase aus Glukoseoxidase besteht und es sich bei dem Elektronenakzeptor um Kaliumferrizyanid handelt.

12. Biosensor nach Anspruch 6, dadurch gekennzeichnet, daß zumindest die Oxidoreduktase in trockenem Zustand auf der porösen Membran durchgeführt wird.

13. Biosensor nach Anspruch 6, dadurch gekennzeichnet, daß die poröse Membran (42) einen Porendurchmesser von weniger als 3 μm aufweist.

14. Biosensor nach Anspruch 6, dadurch gekennzeichnet, daß das poröse Substrat zuvor mit einem oberflächenaktiven Mittel behandelt wurde.

15. Biosensor nach Anspruch 6, dadurch gekennzeichnet, daß ein Antikoagulans auf der porösen Membran (42) getragen wird.

**Revendications**

1. Biocapteur destiné à mesurer la concentration de substrat d'un échantillon liquide, comprenant une plaque isolante de substrat (19; 26; 32; 36) ayant un système d'électrodes contenant au moins une électrode de travail (20; 27; 33; 37) et une électrode auxiliaire (21; 28; 34; 38), le système d'électrodes étant recouvert d'un substrat (23; 29; 39) perméable aux liquides et contenant un enzyme (24; 30; 40) capable de provoquer une réaction du substrat qui est détectable électrochimiquement à l'aide du système d'électrodes, caractérisé en ce que le substrat (23; 29; 39) est poreux et contient une oxydoréductase (24; 30; 40), le capteur contenant en outre un accepteur d'électrons (25; 31; 41), et l'oxydoréductase et l'accepteur d'électrons étant solubles dans l'échantillon liquide.

2. Biocapteur selon la revendication 1, caractérisé en ce que l'enzyme (24; 30; 40) et/ou l'accepteur d'électrons (25; 31; 41) est sous forme de particules finement divisées.

3. Biocapteur selon la revendication 1, caractérisé en ce que le substrat poreux porte à la fois l'oxydoréductase (24; 30; 40) et l'accepteur d'électrons (25; 31; 41).

4. Biocapteur selon la revendication 1, caractérisé en ce que l'électrode de travail (20; 27; 33; 37) contient du platine et l'électrode auxiliaire (21; 28; 34; 38) contient du platine ou de l'argent-chlorure d'argent.

5. Biocapteur selon la revendication 1, caractérisé en ce que le système d'électrodes est composé d'une électrode de travail (20; 27; 33; 37), d'une électrode auxiliaire (21; 28; 34; 38) et d'une électrode de référence (22; 35; 43).

6. Biocapteur selon la revendication 1, caractérisé en ce que le substrat poreux est une membrane hydrophile poreuse.

7. Biocapteur selon la revendication 1, caractérisé en ce que l'accepteur d'électrons est l'oxygène.

8. Biocapteur selon la revendication 1, caractérisé en ce que l'enzyme (24; 30; 40) est choisi dans le groupe qui comprend les oxydases de glucose, d'alcool, de xanthine et de cholestérol.

9. Biocapteur selon la revendication 1, caractérisé en ce qu'un colorant oxydant (25; 31; 41) travaillant en conjugaison avec l'oxydoréductase est utilisé comme accepteur d'électrons.

10. Biocapteur selon la revendication 9, caractérisé en ce qu'un composé sélectionné dans le groupe qui comprend le ferricyanure de potassium, la p-benzoquinone, le 2,6-dichlorophénol-indophénol, le bleu de méthylène, le méthosulfate de phénazine et le β-naphtoquinone-4-sulfonate de potassium est choisi comme colorant oxydant (25; 31; 41) travaillant en conjugaison avec l'oxydoréductase.

11. Biocapteur selon la revendication 9, caractérisé en ce que l'oxydoréductase est la glucosoxydase et l'accepteur d'électrons est le ferricyanure de potassium.

12. Biocapteur selon la revendication 6, caractérisé en ce que l'oxydoréductase au moins est portée par la membrane poreuse, à l'état sec.

13. Biocapteur selon la revendication 6, caractérisé en ce que la membrane poreuse (42) a un diamètre de pores qui ne dépasse pas 3 μm.

14. Biocapteur selon la revendication 6, caractérisé en ce que le substrat poreux a été préalablement traité par un agent tensioactif.

15. Biocapteur selon la revendication 6, caractérisé en ce qu'un anticoagulant est porté sur la membrane poreuse (42).

FIG. 1

FIG. 2

FIG.3

FIG. 4

FIG. 5

response current (µA)

glucose concentration (mg/dl)

FIG. 6

FIG. 7

FIG.8

34

32

33

35

FIG.9

42

39

40

41

36

38    37    43